# EUROPEAN PATENT SPECIFICATION

(11) **EP 0 585 027 B1**
(45) Date of publication and mention of the grant of the patent: **01.04.1998**
(21) Application number: 93306445.3
(22) Date of filing: 16.08.1993
(51) Int. Cl.: A61K 31/20

(54) **Use of fatty acids for treating abnormal tissue calcification**
Verwendung von Fettsäuren zur Behandlung von abnormalen Verkalkung des Gewebes
Utilisation d'acides gras dans le traitement de la calcification anormale des tissus

(30) Priority: 21.08.1992 GB 9217781
(43) Date of publication of application: 02.03.1994
(73) Proprietor: SCOTIA HOLDINGS PLC, Guildford, Surrey GU3 1NA (GB)
(72) Inventor: Horrobin, David Frederick, c/o Scotia Pharm. Ltd., Guildford, Surrey GU1 1BA (GB); Reynolds, Brenda Elizabeth, c/o Scotia Pharm. Ltd., Guildford, Surrey GU1 1BA (GB)
(74) Representative: Farwell, William Robert

(56) References cited:
- EP-A- 0 517 425
- WO-A-87/06463
- US-A- 4 816 483
- THE JOURNAL OF UROLOGY vol. 149, no. S4 , May 1993 page 499A BUCK A.C. ET AL. 'THE PREVENTION OF EXPERIMENTAL NEPHROCALCINOSIS WITH EICOSAPENTAENOIC ACID EPA AND EVENING PRIMROSE OIL GLA'
- THE JOURNAL OF UROLOGY vol. 149, no. S4 , May 1993 page 441A BURGESS N.A. ET AL. 'THE INFLUENCE OF DIETARY EICOSAPENTAENOIC AND GAMMA-LINOLENIC ACID ON ANIMAL MODELS OF RENAL STONE DISEASE'
- CURRENT OPINION IN GASTROENTEROLOGY vol. 8, no. 2 , 2 April 1992 pages 314 - 325 R.J. MALIAKKAL ET AL. 'RECENT ADVANCES IN MEDIUM-CHAIN TRIGLYCERIDES AND FISH OIL'
- THE JOURNAL OF UROLOGY vol. 146, no. 1 , 1991 pages 188 - 194 A.C. BUCK ET AL. 'THE PROTECTIVE ROLE OF EICOSAPENTAENOIC ACID (EPA) IN THE PATHOGENESIS OF NEPHROLITHIASIS'

## Description

### FIELD OF INVENTION

This invention relates to fatty acid treatments, and in particular to prevention and/or treatment of abnormal tissue calcification.

### FATTY ACIDS

The pathways of conversion of the main series of polyunsaturated fatty acids in the body are as in Table 1 below:

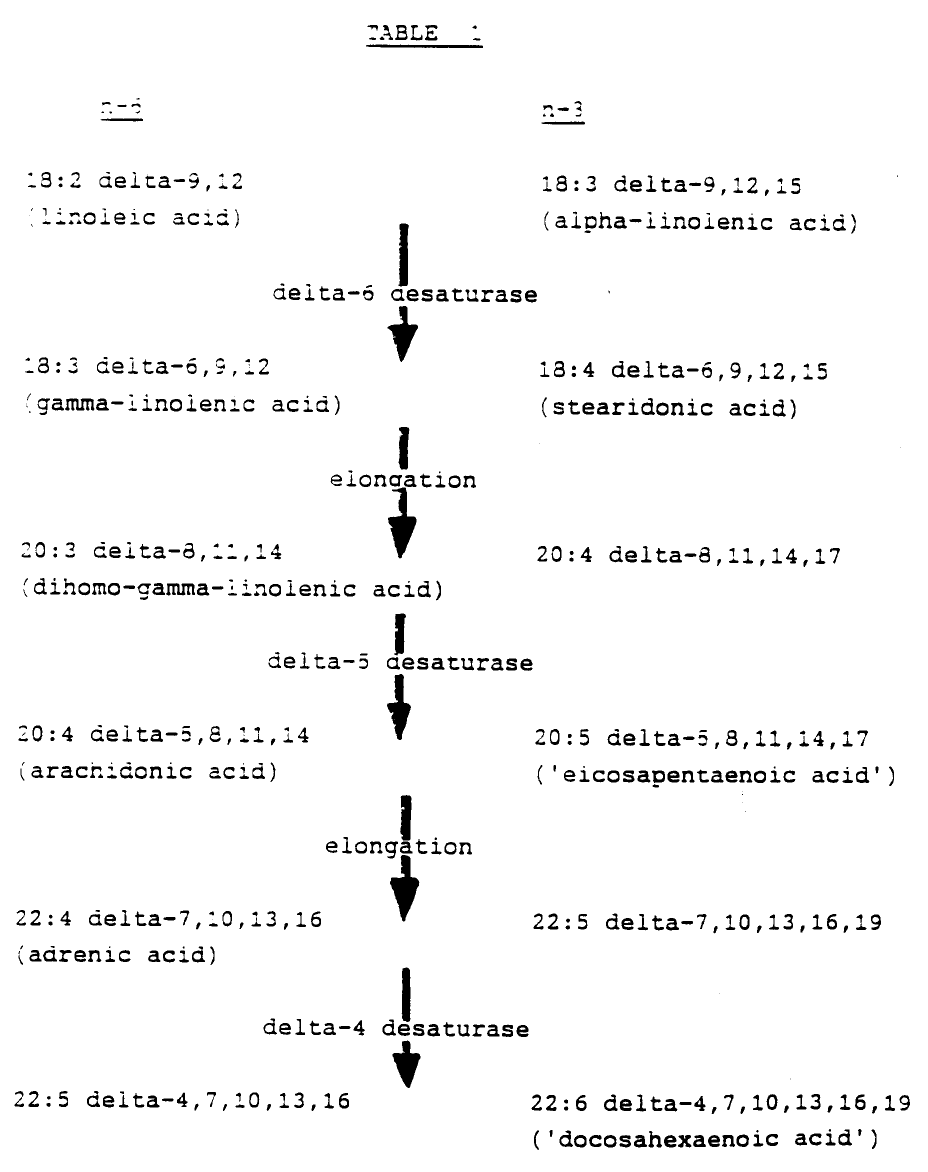

The above pathways are not normally reversible nor, in man, are n-3 and n-6 series acids interconvertible.

The acids, which in nature are of the all-cis configuration, are systematically named as derivatives of the corresponding octadecanoic, eicosanoic or docosanoic acids, e.g. delta-9,12-octadecadienoic acid or delta-4,7,10,13,16,19 docosahexaenoic acid, but numerical designations such as, correspondingly, 18:2 n-6 or 22:6 n-3 are convenient. Initials, for example, EPA for the 20:5 n-3 acid (eicosapentaenoic acid) or DHA for the 22:6 n-3 acid (docosahexaenoic acid), are also used but do not serve when n-3 and n-6 acids of the same chain length and degree of unsaturation exist as for example with the 22:5 acids. Trivial names in more or less common use in the n-6 series are as shown. Of the n-3 series only 18:3 n-3 has a commonly used trivial name, alpha-linolenic acid, though the name stearidonic acid is coming into use for the 18:4 n-3 acid and the names eicosapentaenoic acid and docosahexanenoic acid as such are also used. The alpha isomer of linolenic acid was characterised earlier than gamma-linolenic acid and reference in the literature simply to linolenic acid, especially in the earlier literature, is to the alpha-acid.

### ABNORMAL CALCIFICATION, DISCUSSION AND EXPERIMENTAL

Abnormal calcification may occur for a number of reasons, and may be primary causes of or secondary results of specific disease conditions. The deposition of calcium in the soft tissue (ectopic mineralisation) is nearly always pathological and often its cause is unknown.

Where the circulating level of calcium is consistently high, mineralisation of many soft tissues can occur. The tissues affected include blood vessels, cornea, conjunctivae, skin, brain and kidneys. In parathyroid insufficiency for example, calcification occurs in the basal ganglia and in the skin, whereas in primary or secondary hyperparathyroidism, calcification occurs in the blood vessels and periarticular tissues. Tumoral calcinosis is a condition in which ectopic mineralisation is characterised by the presence of large masses of calcium around the large joints. Calcification can occur in tissues affected by acquired connective tissue disorders such as scleroderma and dermatomyositis, the former occuring sometimes in association with Raynaud's phenomena and telangiectases (CRST syndrome).

Radiation damage to brain microvasculature resulting from radiotherapy may lead to necrosis and calcification of surrounding tissue and grey matter.

Chondrocalcinosis is the formation of mineral outside the skeleton, commonly in joints. A number of diseases are associated with such calcium deposition eg. chronic pyrophosphate arthropathy, acute calcific periarthritis (acute synovitis) and osteroarthritis.

Nephrocalcinosis or calcification of the kidney is a very common form of ectopic calcification. The commonest causes are hyperparathyroidism, hypervitaminosis D and chronic interstitial nephritis. Other causes include osteoporosis, sarcoidosis, renal tubular acidosis, the de Toni-Fanconi syndrome and destruction of bone by metastatic carcinoma. It has been disclosed (Current opinion in Gastroenterology, 1992, 8, 314 -325, Maliakkal et al; J. Neurology, 1991, 146, 188-194, Buck et al) that EPA is beneficial in the treatment or prevention of nephrocalcinosis and renal stones.

Because of the widespread occurence of soft tissue calcification in disease conditions, the applicants designed an animal model of ectopic calcification in the kidney to investigate the effects of treatment with 6-desaturated essential fatty acids of both the n-6 and n-3 series. (this example is not part of the invention) 74 female PVG (piebald virol glaxo) rats were divided into 7 groups as described below:-
Group 1 Controls treated with 1.5ml of 10% calcium gluconate daily for 10 days to induce nephrocalcinosis (n = 10);
Group 2 Given eicosapentaenoic acid (EPA) 38mg by gavage daily 4 days prior to and during 10 days of calcium gluconate administration as in 1 above (n = 10);
Group 3 Given gammalinolenic acid (GLA) 66mg plus EPA 60mg as in 2 above (n = 10);
Group 4 Given gammalinolenic acid 83mg per day as in 2 above (n = 10);
Group 5 Given 1ml sunflower oil per day as in 2 above (n = 10);
Group 6 Given 1ml olive oil per day as in 2 above (n = 10);
Group 7 Given only an i.p. injection of saline for 10 days (n = 14).

The presence of calcification at day 15 was assessed qualitatively by histology using von kossa and alizarin staining and quantitatively by wet chemical spectroscopic analysis. The results are summarised below:-

| Group | Calcium in Kidney mg/g dry weight | Significance |
|---|---|---|
| 1. Control (treated) | 0.83 ± 0.51 | |
| 2. EPA | 0.10 ± 0.13 | *** |
| 3. GLA plus EPA | 0.19 ± 0.19 | *** |
| 4. GLA | 0.21 ± 0.16 | *** |
| 5. Sunflower Oil | 1.30 ± 0.97 | |
| 6. Olive Oil | 1.12 ± 0.55 | |
| 7. Control (untreated) | 0.15 ± 0.04 | *** |

These results indicate that both GLA and EPA individually or in combination can significantly decrease calcification of the kidney in rats even when circulating calcium levels are extremely high.

### THE INVENTION

Based on the above, the invention in one aspect lies in the use of (i) GLA and/or its essential fatty acid (EFA) metabolites, and in particular the rapidly produced dihomo-gamma-linolenic acid (DGLA) and/or (ii) EPA and/or its precursors, stearidonic acid and 20:4 n-3, and metabolites, docosahexaenoic acid (DHA) or docosapentaenoic acid (22:5 n-3) for prevention and/or treatment of abnormal calcification of any tissue in both animals and humans. The abnormal calcification may be the primary cause of one of the diseases previously reviewed, or may result from another disease.

Alternatively, the invention may be regarded as lying in a method of, or preparation of a medicament for, treating or preventing abnormal calcification of any tissue in humans or in animals by administering (i) GLA and/or its EFA metabolites, and in particular the rapidly produced DGLA and/or (ii) EPA and/or its precursors, stearidonic acid and 20:4 n-3, and metabolites, docosahexaenoic acid (DHA) or docosapentaenoic acid (22:5 n-3).

As noted, GLA in the body is very rapidly converted to dihomo-gamma-linolenic acid (DGLA); DGLA therefore has a very similar effect to GLA.

As discussed further below the essential fatty acids may be used as such or in any appropriate form, including but not limited to triglyceride, diglyceride, monoglyceride, free fatty acid, any appropriate ester, any appropriate salt including the lithium, sodium, potassium, zinc, magnesium or other salt, phospholipid, amide or any other pharmacologically acceptable form.

The preferred dose range is from 0.01 to 1,000 mg/kg/day, more preferably from 0.5 to 100 mg/kg/day, very preferably from 2 to 30 mg/kg/day of GLA or DGLA, and medicaments are readily prepared in dosage unit form to administer such amounts (related to a 70 kg human adult).

### ROUTES OF ADMINISTRATION

Oral, parenteral (sub-cutaneous, intramuscular, intravenous or by any other appropriate route), enteral, topical in the form of appropriate EFA-containing ointments, creams, lotions, patches, etc. vaginal or rectal are among suitable routes of administration.

### DERIVATIVES OF EFAs

As indicated above, the acids may be used as such or as pharmaceutically acceptable and physiologically equivalent derivatives as, for example, detailed herein for GLA and DGLA, and reference to any of the acids is to be taken as including reference to the acids when in the form of such derivatives. Equivalence is demonstrated by entry into the pathway quoted herein, as evidenced by effects corresponding to those of the acids themselves or their natural glyceride esters. Thus, indirect identification of useful derivatives is by their having the valuable effect in the body of the acid itself, but conversion can be shown directly by gas chromatographic analysis of concentrations in blood, body fat, or other tissue by standard techniques, for example those of Pelick et al, page 23, "Analysis of Lipids and Lipoproteins" Ed Perkins, American Oil Chemists Society, Champaign, Illinois, U.S.A.

In outline the method is suitably that plasma samples (1 ml) are extracted with chloroform:methanol (2:1). The extract is filtered through sodium sulphate, evaporated to dryness, and taken up in 0.5 ml chloroform:methanol. The lipid fractions are separated by thin layer chromatography or silica gel plates. The phospholipid fraction, taken to reflect essential fatty acid contents most sensitively, is methylated using boron trifluoride-methanol. The resulting methyl esters of the fatty acids are separated and measured using a Hewlett-Packard 5880 gas chromatograph with a six foot column packed with 10% Silar on Chromosorb WAW 106/230. The carrier gas is helium (30 ml/min). Oven temperature is programmed to rise from 164°C to 190°C at 2°C/min. Detector temperature is 220°C and injector temperature 200°C. Retention times and peak areas are automatically computed by Hewlett-Packard Level 4 integrator. Peaks are identified by comparison with standard fatty acid methyl esters.

### DIETARY COMPOSITIONS

The invention is chiefly described in terms of methods of treatment and in the use of GLA or its metabolites and/or EPA or its metabolites in the preparation of pharmaceutical compositions, but it will be understood that the gamma-linolenic and other EFAs, being in the nature of dietary supplements, can be incorporated in a dietary margarine or other foodstuff and such are to be understood as within the term pharmaceutical composition or medicament herein (including the claims) when for the purposes set out.

If desired, pharmaceutical compositions may be produced for use in the invention by associating the natural or synthetic acids, as such or as derivatives, with an acceptable pharmaceutical vehicle. It is, however, at present convenient to provide at least GLA in the form of an available oil having a high GLA content, hence reference to "oils" herein.

One source of oils currently available is the seed of evening primrose species such as Oenothera biennis L. and Oenothera lamarckiana, the oil extract therefrom containing about 8% GLA and about 72% linoleic acid in the form of their glycerides, together with other glycerides (percentages based on total fatty acids). Other sources of GLA are borage species such as Borago officinalis which provide a richer source than Oenothera oil. Oils from the seeds of members of the Ribes family are also often rich in GLA. Recent studies on fungi which can be cultivated by fermentation promise a fungal oil source. Some algae also produce GLA and may be harvested or cultured. Synthesis is also possible.

The oil is extracted from the seed by one of the conventional methods of extraction such as cold pressure, screw pressure after partially cooking the seed, or solvent extraction.

Fractionation of a typical sample of evening primrose oil as used in the work reported herein in the form of methyl esters shows the relative proportions:

| | |
|---|---|
| Palmitate | 6.15 |
| Stearate | 1.6 |
| Oleate | 10.15 |
| Linoleate | 72.6 |
| Gamma-linolenate | 8.9 |

The seed oil extracts referred to above can be used as such or can, for example, if desired, be fractionated to yield an oily composition containing the triglycerides of gamma-linolenic and linoleic acids as the main fatty acid components, the gamma-linolenic acid content being, if desired, a major proportion. Seed oil extracts appear to have a stabilising effect upon DGLA if present.

GLA, EPA and the other EFAs or their derivatives or metabolites may be produced by any suitable method including chemical synthesis, fermentation (microbial or algal), extraction from animal, botanical or other source, agronomy or other methods as appropriate.

### PHARMACEUTICAL PRESENTATION

As mentioned briefly above, the compositions are conveniently in a form suitable for oral, topical, parenteral or other route of administration in a suitable pharmaceutical vehicle, well known generally for any particular kind of preparation. Thus, for example, tablets, capsules, ingestible liquid or powder preparations can be prepared as required, and topical preparations also when the gamma-linolenic acid or other acids are absorbed through the skin. Injectable solutions of hydrolysed Oenothera or other oil may be prepared using albumin to solubilise the free acid. Emulsions and salts can also be administered by infusion or injection.

Advantageously, a preservative is incorporated into the preparation. Alpha-tocopherol in a concentration of about 0.1% by weight has been found suitable for the purpose and is one of a number of possible stabilisers well known in the field and including also for example ascorbyl palmitate and stearate.

It will be understood that the absolute quantity of active materials present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The composition may also be in the form of whips, emulsions, suspensions, pessaries, suppositories, transdermal devices or any other appropriate forms.

### EXAMPLES

The following are examples of compositions and their administration for the purposes discussed herein.
1. Administration of 100 mg to 2,000 mg of GLA per day in the form of soft or hard gelatin capsules or tablets providing:
   a. 40 to 80 mg per capsule of GLA in the form of evening primrose oil.
   b. 50-150 mg per capsule of GLA in the form of borage, blackcurrant, fungal or other appropriate oil.
   c. 100-150 mg GLA per capsule in the form of triglyceride GLA, or any appropriate salt of GLA, such as the lithium or calcium or magnesium or zinc or potassium salts.
2. Administration of 100 mg to 2,000 mg of EPA per day in the form of soft or hard gelatin capsules or tablets providing:
   a. 90 to 180 mg per capsule of EPA in the form of cold water marine fish oil.
   b. 200 to 600 mg per capsule of EPA in the form of an enriched EPA oil.
   c. 400 to 1,000 mg of EPA per capsule in the form of triglyceride EPA or any appropriate salt of EPA such as the lithium or calcium or magnesium or zinc or potassium salts.
3. Administration of DGLA in a dose of 100 mg to 2,000 mg per day in the forms of 1c above.
4. Administration of GLA or DGLA in association with EPA, with or without DHA, for example as 40 to 80 mg GLA per capsule in the form of evening primrose oil together with 10 mg to 100 mg per capsule of EPA in the form of cold water marine fish oil.
5. Administration of GLA or DGLA in the form of a soluble powder or effervescent granule formed from any appropriate salt of GLA as in 1c above and excipients such as citric acid monohydrate, sodium bicarbonate or other dibasic acids such as tartaric or maleic acid plus sweeteners such as sucrose or sorbitol and flavourings.
6. Administration of GLA or DGLA in the form of liquid evening primrose, borage or other appropriate oil as the oil itself or as a whip or emulsion prepared with appropriate flavours and stabilisers.
7. Administration of EPA or DHA in any appropriate chemical form, microencapsulated using starch, gelatin, gum arabic or other appropriate formulation.
8. Administration of GLA in the form of pessaries, suppositories, skin patches or any other appropriate route.

## Claims

1. Use of (i) gammalinolenic acid (GLA) and/or its essential fatty acid (EFA) metabolites, and in particular the rapidly produced dihomo-gamma-linolenic acid (DGLA) and/or (ii) Eicosapentaenoic acid (EPA) and/or its precursors, stearidonic acid and 20:4 n-3, and metabolites, docosahexaenoic acid (DHA) or docosapentaenoic acid (22:5 n-3) wherein the essential fatty acids are used as such or in salt or other pharmacologically acceptable form, in the preparation of a medicament for treating or preventing abnormal calcification of soft tissue of blood vessels, cornea, conjunctivae, skin and brain in humans or in animals.

2. A use according to claim 1, wherein the medicament comprises a dose range of the or each essential fatty acid suited to administration of from 0.01 to 1,000 mg/kg/day, preferably from 0.5 to 100 mg/kg/day, more preferably from 2 to 30 mg/kg/day.

3. A use according to claim 1 or 2, wherein the medicament is presented in unit dosage form.

## Patentansprüche

1. Verwendung von (i) Gammalinolensäure (GLA) und/oder ihren unentbehrlichen Fettsäure(EFA)-Metaboliten und insbesondere der schnell produzierten Dihomo-Gammalinolensäure (DGLA), und/oder (ii) EicosapentaenoicSäure (EPA) und/oder ihrer Vorgänger, Stearidonsäure (20:4 n-3) und Metaboliten, docosahexaenoic Säure (DHA) oder docosapentaenoic Säure (22:5 n-3), in denen die unentbehrlichen Fettsäuren als solche oder in Salz oder einer anderen, pharmakologisch akzeptablen Form bei der Herstellung eines Medikaments zur Behandlung oder Verhinderung von abnormer Verkalkung von weichem Gewebe von Blutgefäßen. Kornea, Konjunktivä, Haut und Gehirn in Menschen oder in Tieren benutzt werden.

2. Eine Verwendung nach Anspruch 1, wobei das Medikament einen Dosisbereich der oder jeder unentbehrlichen Fettsäure umfaßt, der für die Verabreichung von 0,01 bis 1000 mg/kg/Tag, möglichst von 0,5 bis 100 mg/kg/Tag, und noch besser von 2 bis 30 mg/kg/Tag geeignet ist.

3. Eine Verwendung nach Anspruch 1 oder 2, wobei das Medikament in Einheitsdosierungsform präsentiert wird.

## Revendications

1. Utilisation de (i) acide gamma-linolénique (GLA) et/ou de ses métabolites acides gras essentiels, et en particulier de l'acide dihomogamma-linolénique (DGLA), rapidement produit, et/ou de (ii) acide éicosapentaénoïque (EPA) et/ou de ses précurseurs, l'acide stéaridonique et le 20:4 n-3, et de ses métabolites, l'acide docosahexaénoïque (DHA) ou l'acide docosapentaénoïque (22:5 n-3),où les acides gras essentiels sont utilisés en l'état ou sous forme d'un sel ou sous une autre forme acceptable d'un point de vue pharmacologique, pour préparer un médicament destiné à traiter ou prévenir une calcification anormale des tissus mous, des vaisseaux sanguins, de la cornée, de la conjonctive, de la peau et du cerveau, chez l'homme ou l'animal.

2. Utilisation selon la revendication 1, dans laquelle le médicament comprend un intervalle posologique de l'acide gras essentiel ou de chacun des acides gras essentiels convenant à une administration de 0,01 à 1000, de préférence de 0,5 à 100, et plus particulièrement de 2 à 30 mg/kg/jour.

3. Utilisation selon la revendication 1 ou 2, dans laquelle le médicament se présente sous une forme posologique unitaire.
